# EUROPEAN PATENT SPECIFICATION

(11) **EP 1 390 759 B1**
(45) Date of publication and mention of the grant of the patent: **23.03.2005**
(21) Application number: 02730840.2
(22) Date of filing: 31.05.2002
(51) Int. Cl.: G01N 33/86

(54) **ASSAY METHOD FOR PLATELET-ACTIVATING FACTOR**
VERFAHREN FÜR DIE BESTIMMUNG VON PLÄTTCHEN AKTIVIERENDEM FAKTOR
PROCEDE DE DOSAGE DU FACTEUR D'ACTIVATION DES PLAQUETTES

(30) Priority: 31.05.2001 JP 2001164982
(43) Date of publication of application: 25.02.2004
(73) Proprietor: Daiichi Suntory Pharma Co., Ltd., Tokyo 102-0083 (JP)
(72) Inventor: KANAI, Yasushi, Ashikaga-shi, Tochigi 326-0824 (JP); HAYASHI, Yujiro, Tatebayashi-shi, Gunma 374-0038 (JP); ONUMA, Norio, Tatebayashi-shi, Gunma 374-0018 (JP); MIYAZAKI, Hiroshi, Kawasaki-shi, Kanagawa 213-0021 (JP)
(74) Representative: HOFFMANN - EITLE
(86) International application number: PCT/JP2002/005369
(87) International publication number: WO 2002/097435

(56) References cited:
- EP-A- 0 345 100
- RAMESHA, C. ET PICKETT, W.C.: "Human neutrophil platelet-activating factor molecular heterogeneity in unstimulated and ionophore-stimulated cells" BIOCHIMICA ET BIOPHYSICA ACTA, vol. 921, no. 1, 1987, pages 60-66, XP009010287 cited in the application
- YAMADA, K. ET AL: "Highly sensitive gas chromatographic mass spectrometric method for the determination of platelet-activating factor in human blood" JOURNAL OF CHROMATOGRAPHY BIOMEDICAL APPLICATIONS, vol. 433, 1988, pages 243-247, XP001147421 cited in the application
- FISCHER, A.L. ET AL: "Determination of MK-287, a new platelet-activating factor antagonist, in plasma and serum by gas chromatography chemical ionization mass spectrometry" BIOLOGICAL MASS SPECTROMETRY, vol. 20, no. 7, July 1991 (1991-07), pages 408-414, XP009010310
- KARASAWA, K. ET AL: "Radioimmunassay for platelet-activating factor" LIPIDS, vol. 26, no. 12, 1991, pages 1126-1129, XP009010288 USA
- NOMIKOS, T. N. ET AL: "Determination of platelet-activating factor by HPLC with fluorescence detection" JOURNAL OF LIQUID CHROMATOGRAPHY & RELATED TECHNOLOGIES, vol. 22, no. 9, May 1999 (1999-05), pages 1331-1341, XP009010284

## Description

### FIELD OF THE INVENTION

The present invention relates to an assay method for PAF whereby PAF is assayed in a highly effective and specific manner based on selectively extracting and purifying PAF from a biological sample, followed by a gas chromatography-mass spectrometry (GC-MS) method and a liquid chromatography-mass spectrometry (LC-MS) method. The method of the invention may be used to measure PAF concentrations in biological samples for elucidating the role of PAF in various pathological conditions. In addition, it is expected to facilitate diagnosis of various diseases associated with fluctuation in PAF, while also contributing to evaluation of therapeutic effects on the above diseases.

### PRIOR ART

PAF was discovered in 1972 as a platelet-activating humoral factor which is released from IgE-sensitized rabbit basophils by antigenic stimulation, and the structure of PAF derived from rabbit basophils was determined in 1972 to be 1-O-alkyl-2-acetyl-sn-glycero-3-phosphocholine. It has ever since being attempted to detect and assay PAF from various biological samples, and at the present stage the various molecular species have been identified in the PAF family.

PAF is expressed by the following formula: 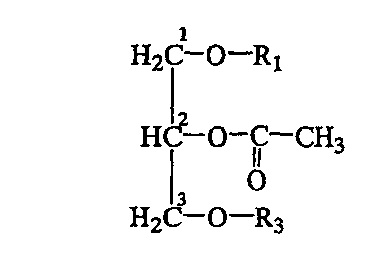 (where R₁ is a saturated or unsaturated noncyclic hydrocarbon group or an acyl group derived from a saturated or unsaturated fatty acid, and R₃ is phosphocholine, phosphoethanolamine, phosphomonomethylethanolamine or phosphodimethylethanolamine). Hence, PAF is a kind of phospholipid, having a structure wherein a saturated or unsaturated noncyclic hydrocarbon group or an acyl group derived from a saturated or unsaturated fatty acid is bonded to the oxygen atom at Cl of the glycerol skelton to form an ether, ester or vinylether bond, an acetyl group is bonded to it at C2, and phosphocholine, phosphoethanolamine, phosphomonoethylethanolamine or phosphodimethylethanolamine is bonded to it at C3. The saturated or unsaturated noncyclic hydrocarbon groups or acyl groups derived from saturated or unsaturated fatty acids at the C1 carbon are known to have 12-18 carbon atoms. It has been known that each of these molecular species exhibits its different biological activity, and 1-0-hexadecyl-2-acetyl-sn-glycero-3-phosphocholine (C₁₆-PAF) exhibits the strongest biological activity among these molecular species. The principal physiological functions of PAF are platelet activation, leukocyte migration and activation, vascular permeability promotion and the like, and it is thought to be a contributing factor in allergic symptoms such as bronchial asthma, endotoxin shock and various inflammatory conditions. In order to clearly determine whether or not PAF is involved in these symptoms, it is indispensable to measure PAF concentrations in biological samples taken from patients with these symptoms, and then to examine fluctuations in those concentrations. However, PAF assay has been difficult due to its extremely low concentrations of PAF in biological samples and the coexistence of high concentrations of biological components with structures very similar to PAF, and therefore at the current time, the involvement of PAF in various diseases has not yet been elucidated.

Methods for PAF assay which have been hitherto reported have been classified mainly into three categories: biological activity assay methods, immunological assay methods and physicochemical assay methods such as GC-MS and LC-MS methods ("Kesshoban Kasseika Inshi" [Platelet-Activating Factor], Gendai Kagaku, Special Edition 17, 25-34, 1989). Almost all biological activity assay methods are based on platelet aggregation. However, since biological components besides PAF also exhibit platelet aggregation, while differences in the molecular species of PAF give rise to different platelet aggregation activities, it is currently not possible to specifically determine PAF by biological activity assay methods. With immunological assay methods, since phosphatidylcholine (PC) and lysophosphatidylcholine (LysoPC) which have structures very similar to PAF are also coexisted in the biological samples with high concentrations and these phospholipids give rise to the cross react with the antibody against PAF, precise and accurate assay of PAF in biological samples has not been achievable by the use of immunological assay methods.

In regard to physicochemical assay methods, Ramesha C.M. et al. have developed a highly sensitive method for quantifying PAF by GC-MS (Biomed. Environ. Mass Spectrom. 13, 107-111, 1986). They hydrolyzed the phosphocholine group at C3 of C₁₆-PAF using phospholipase C, and then introduced a pentafluorobenzoyl group into the hydroxyl group of the resulted glycerol compound, and subjected to GC-MS analysis. Because this method allows detection of PAF at high sensitivity, many researchers have been used their method for quantifying PAF by GC-MS.

However, even when applying this GC-MS method to the assay of C₁₆-PAF concentrations in biological samples, the C₁₆-PAF concentrations in biological samples such as blood reported so far have been much higher than the actual levels because the contaminating phospholipids cannot be completely removed. Thus, it is currently the situation that no practical method has been developed for measuring PAF concentrations in biological samples, and therefore development of a PAF assay method with high sensitivity and high specificity has been desired.

### SUMMARY OF THE INVENTION .

The present invention solves the problems described above, and its object is to provide a highly sensitive, highly specific method for the quantitation PAF in any samples suspected of containing even PAF analogs, such as biological samples (hereinafter these will be collectively referred to simply as "biological samples"), by the highly reproducible and reliable GC-MS method and LC-MS method.

More specifically, the invention provides a solvent system that extracts PAF from biological samples with maximum efficiency for analysis.

The invention further provides a method for efficiently removing PAF analogs from biological samples by using a silica gel column and specific washing and eluting solutions.

The invention still further provides a method for hydrolyzing selectively PAF in a biological sample coexisting LysoPC which is not removed by the aforementioned silica gel column using phospholipase C, and reseparating the resulted glycerol compound from the unchanged LysoPC, in the case when a GC-MS method is employed in the analysis.

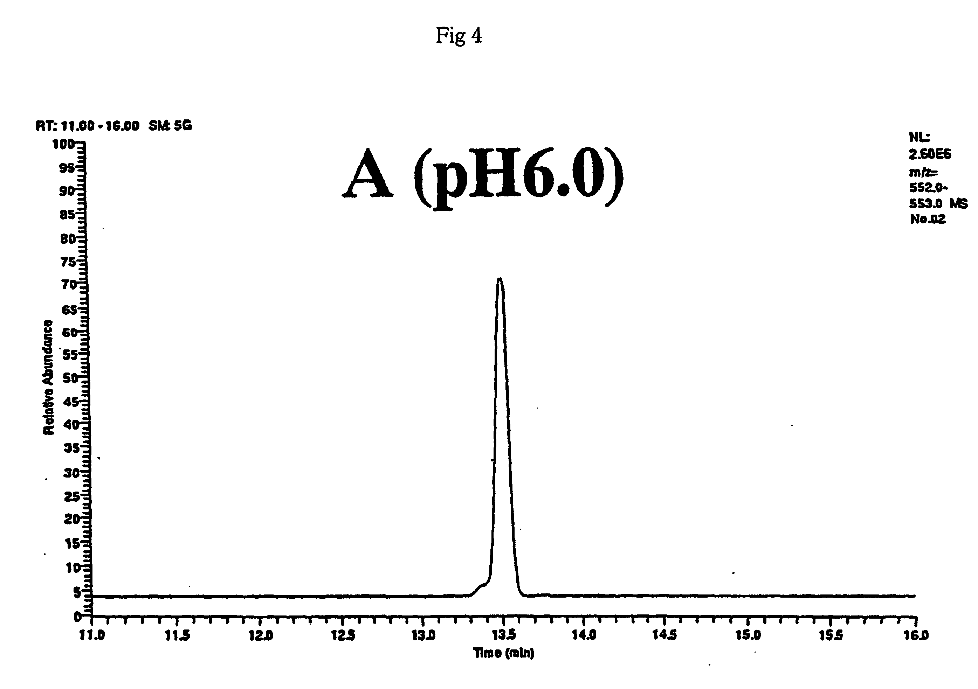

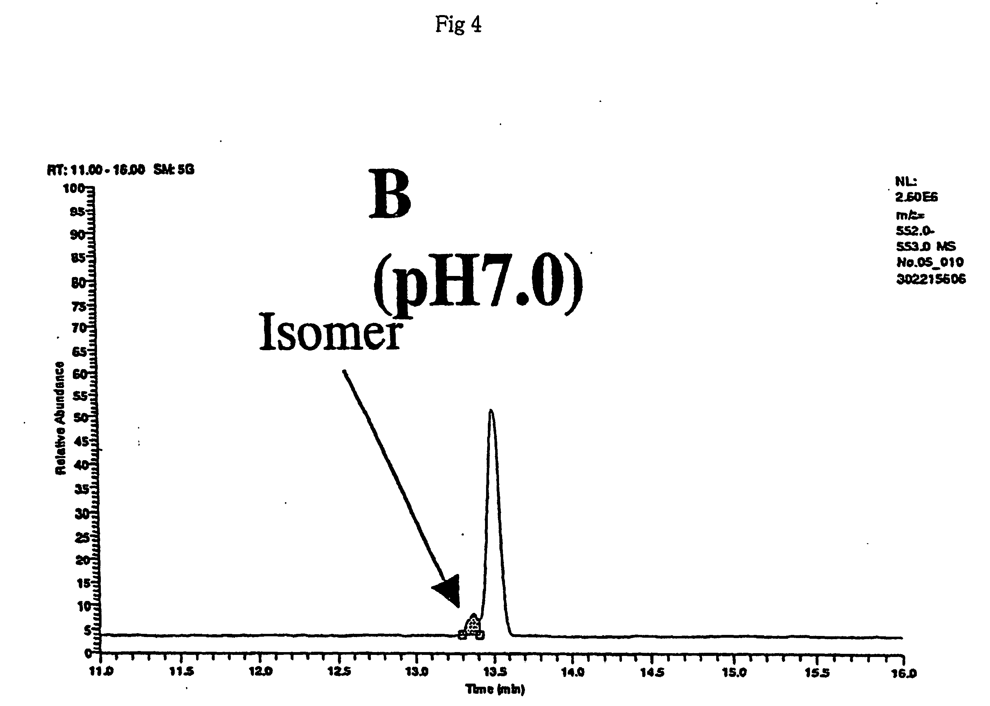

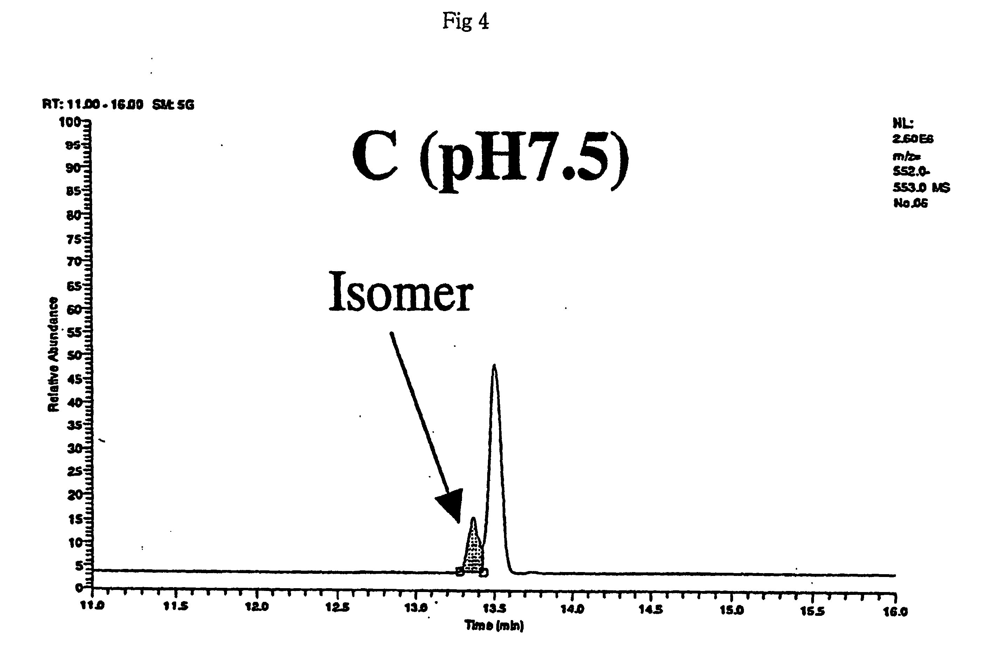

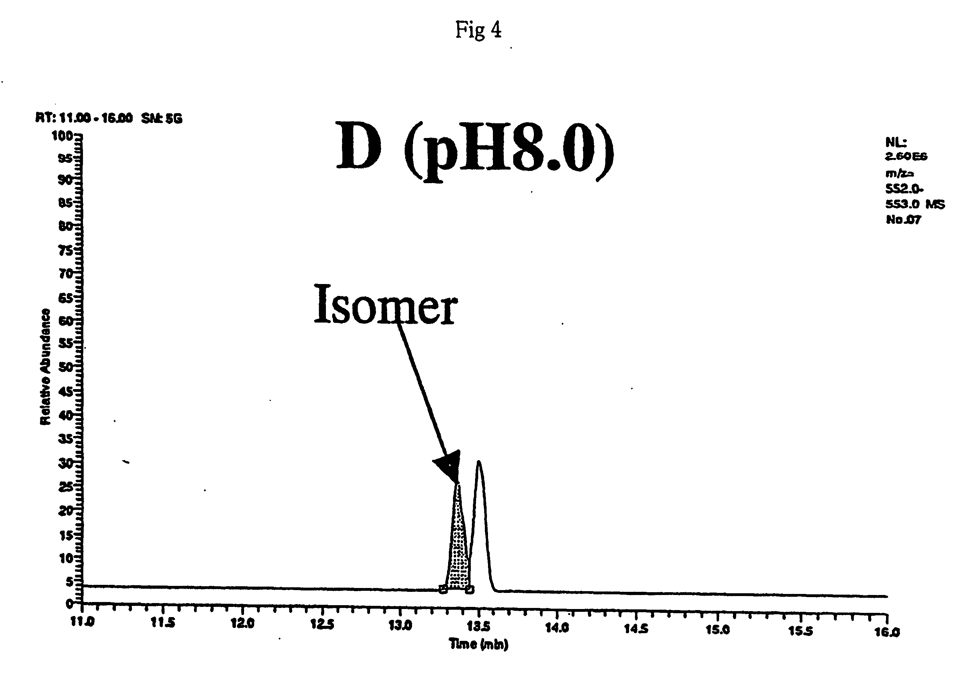

The invention still further provides a method for hydrolysis reaction of PAF with phospholipase C, so that in the pentafluorobenzoyl action for analysis of the glycerol compound by gas chromatography, the resulted derivative does not include the isomers shown in Figs. 4-B, C and D.

The invention still further provides a LC-MS method for measuring PAF in a biological sample rapidly and accurately with a high-sensitivity without any further pre-treatment of the biological sample after removal of PAF analogs from the biological sample by use of the above mentioned silica gel column with the specific combination of the washing and eluting solvents.

### BRIEF DESCRIPTION OF THE DRAWINGS

Fig. 1. Recordings of gas chromatography-selected ion monitoring (GC-SIM) verifying the uniformaty of the PAF peak obtained from the human blood sample of Example 3 by treatment of it with rhPAF-AH
Fig. 2. Recordings of GC-SIM comparing the extraction method of the invention according to Example 4 with that of Bligh & Dyer (methanol/chloroform/water).
Fig. 3. Recordings of GC-SIM comparing the purification method of the invention according to Example 5 using a normal phase-solid column with that of Weintraub, S.T. et al.
Fig. 4. Recordings of GC-SIM showing production of the isomer by the PH dependency of incubation media for hydrolyzing PAF with phospholipase C according to Example 6.
Fig. 5. Calibration curve for quantifying PAF concentration of human blood in the range of 0 - 181 pg/ml, and that for estimating the endogenous PAF blood level in the range of 0 - 20.2 pg/ml according to Example 7.
Fig. 6. Recordings of liquid chromatography-selected reaction monitoring measuring PAF in human blood according to Example 8.

### DETAILED DESCRIPTION

The present invention may be applied as a PAF assay method for biological activity assay methods, immunological assay methods or physicochemical assay methods, but the GC-MS methods and LC-MS methods are preferred due to the highest sensitivity and specificity.

In order to assay PAF in a biological sample it is necessary first to efficiently extract the PAF from the sample and then adequately separate out the coexisted PAF analogs.

Specifically, when extracting the PAF from the biological sample, it is assumed that large amounts of biological components besides PAF may also be coexisted. Therefore, in the step of further purification of the extract with a solid-phase extraction column or the like, optimization of washing and eluting conditions is need to separate PAF from PAF analogs. For example, the method of Yamada K. et al. described in J. Chromatogr. 433, 243-247, 1988, adopts the method of Bligh & Dyer (Can. J. Biochem. 37, 911-917, 1959), which has been exclusively used in the prior art as a phospholipid extraction method, where methanol is added to and mixed with blood, and after centrifugation, chloroform is added to the supernatant and the lower layer is separated out. A normal solid-phase extraction column is then used to purify the PAF using chloroform/methanol/water-based washing and eluting solutions. However, the present inventors have shown that numerous biological components besides PAF are extracted by this method, and that inadequate separation and purification of PAF from its analogs results in overestimation of human blood PAF concentrations to be 10-30 pg/mL, which are higher than the actual levels.

Thus, the first step was to actively investigate solvent systems for efficient extraction of PAF from biological samples, and purification methods for further purification of the extracts to completely remove PAF analogs.

The present inventors have further shown that, in the case of quantitation of PAF by a GC-MS method, the precision and accuracy of a PAF assay are greatly affected by the reaction conditions for phospholipase C hydrolysis of PAF which has been extracted and purified from a biological sample. Most conventional methods have been set the reaction pH to neutral or in the weakly alkaline range. However, it has been found that when the reaction solution pH is neutral or weakly alkaline, isomers are produced during the hydrolysis of PAF, and this not only decreases the yield of PAF glycerol compound, but since isomers of biological components besides PAF are also produced, numerous interference peaks appear, further complicating the analysis of PAF.

It has also been found that phospholipase C treatment also produces the same glycerol compounds from PAF analogs (phospholipids having the phosphocholine at the C3 position replaced with phosphoethanolamine, phosphomonoethylethanolamine or phosphodimethylethanolamine) as are obtained from PAF, and that these also have a significant influence on the measuring precision, such as resulting in overestimation of the PAF concentrations.

The present invention was completed on the basis of these findings, and comprises the following steps.
(1) Mixing a sample whose PAF concentration is to be assayed (for example, a sample suspected of also containing PAF analogs, and especially a biological sample) with a water-soluble organic solvent, such as tetrahydrofuran, as a entirely different solvent from that used for the method of Bligh & Dyer, and accomplishing separation by centrifugation or the like;
(2) Adding a water-insoluble organic solvent such as ethyl acetate to the supernatant obtained from the separation, and performing separation by centrifugation or the like to extract the PAF in the supernatant with a high yield;
(3) Loading the extract into a solid-phase extraction column of silica gel or the like (preferably a normal solid-phase extraction column) to purify PAF from the extract, and then performing washing and elution with a hexane/2-propanol/water mixture to purify the PAF and separate it from the coexisting PAF analogs;
(4) When a GC-MS method is employed for the quantitation, hydrolyzing the purified PAF with phospholipase C, preferably under conditions in which LysoPC is not hydrolyzed;
(5) Separating the hydrolyzed PAF from the unhydrolyzed compounds (for example, the unhydrolyzed LysoPC);
(6) Converting the glycerol compound produced by the hydrolysis to a pentafluorobenzoyl derivative and then quantifying its amount by GC-MS;
(6') When a LC-MS method is employed for the quantitation, the purified PAF from step (3) is subjected to the LC-MS method without further purification; and
(7) If necessary, comparing it with a calibration curve obtained by subjecting a known concentration of PAF to steps (1) to (6), to quantify the amount of PAF in the sample.

As samples to be assayed by the method of the invention there may be mentioned biological samples such as biological fluids (blood, serum, plasma, saliva, urine, bile, pancreatic juice, etc.) and tissues. Since blood is most easily obtainable and useful for disease diagnosis among such samples, examples using blood will be principally referred to in explaining the preferred mode of the invention.

### (1) Extraction of PAF with water-soluble organic solvent

According to the invention, a combination of a water-soluble organic solvent and a water-insoluble organic solvent is used for high-yield extraction of PAF from blood.

Tetrahydrofuran is ideal as the water-soluble solvent because it rapidly inactivates PAF acetylhydrolase in blood and allows complete recovery of the PAF in the supernatant obtained by centrifugation of the sample. The tetrahydrofuran used preferably has the peroxides removed. Alternatively, acetonitrile, 2-propanol, dioxane, acetone, ethanol or the like may be used as the water-soluble organic solvent. The sample and water-soluble organic solvent are mixed in a volume ratio of approximately 1:1-1:10 and stirred at room temperature for about 30 seconds, and then the mixture is centrifuged (for example, 3000 rpm, 15 minutes, 4°C) and the supernatant obtained.

### (2) Extraction of PAF by water-insoluble organic solvent

Since water-soluble blood components except PAF are included in the extract obtained with the water-soluble organic solvent (in the supernatant after centrifugation), a water-insoluble organic solvent is added to remove the water-soluble blood components. Suitable organic solvents include hexane, cyclohexane, xylene, toluene, benzene, diethyl ether, ethyl acetate and the like. When the water-insoluble organic solvent is added to the aforementioned tetrahydrofuran extract and mixed and the mixture is centrifuged, PAF is almost quantitatively recovered in the supernatant, with the water-soluble blood components migrating to the lower layer, thereby allowing separation of PAF from the water-soluble blood components. The water-soluble organic solvent extract and the water-insoluble organic solvent are mixed in a volume ratio of approximately 1:0.5-1:10 and stirred at room temperature for about 30 seconds, and then centrifuged (for example, 3000 rpm, 15 minutes, 4°C) to obtain the PAF extract.

In the combination of the water-soluble organic solvent and water-insoluble organic solvent of the invention, tetrahydrofuran as the water-soluble organic solvent drastically improves the extraction yield of PAF from a sample as compared to the solvent employed in the method of Bligh & Dyer, which is a combination of methanol, chloroform and water, and has been used by the method of Yamada et al. (J. Chromatogr. 433, 243-247, 1988).

### (3) Purification of PAF

The PAF in the extract is then purified with a solid-phase extraction column. The column used is preferably a normal phase-type silica gel column, and for example, the commercially available product Bond Elut SI™ may be used. As a result of investigating column washing solvents and elution solvents, the present inventors have found out that performing washing and elution with a mixture of hexane/2-propanol/water as the column washing solvent and elution solvent is effective for selective purification of PAF. The preferred proportion for n-hexane/2-propanol/water is 1/0.5-1/0.05-0.2 for the washing step and 1/2-20/0.2-2 for the elution step.

Selection of this solvent system allows PAF analogs to be separated in a much more satisfactory manner than by purification of PAF with the chloroform/methanol/water-based washing solution and elution solution used in the method of Yamada K. et al. (J. Chromatogr. 433, 243-247, 1988). In addition, since the supernatant of the water-insoluble organic solvent can be directly loaded into the solid-phase extraction column from step (2) above, the operation efficiency is grately improved.

The extraction and purification method may be used for measurement using biological activity assay methods, immunological assay methods and physicochemical assay methods including LC-MS. For measurement by GC-MS which has superior sensitivity and specificity, the following additional treatment is necessary.

### (4) Hydrolysis of PAF by phospholipase C

The PAF purified by (3) above is treated with phospholipase C in order to hydrolyze the phosphocholine at the C3 position of PAF to convert it into a volatile derivative suitable for GC-MS.

It has been known that a rearrangement reaction occurs between the acetyl group at the C2 position of PAF and the pentafluorobenzoyl group at the C3 position followed by derivatization during this procedure, as shown in Figs. 4-B, C and D, producing isomer. The production of the isomer decreases the peak intensity for PAF and also increases interference peaks derived from biological components, resulting in reduced detection limit and specificity. Consequently, this rearrangement reaction must be minimized. It has been thought that this rearrangement reaction is caused by the step of pentafluorobenzoylation of the glycerol compound produced by hydrolysis of PAF by phospholipase C as reported by Haroldsen P.E. et al. (J. Lipid Res. 32, 723-729, 1991) or the step of purification by normal phase silica gel chromatography as reported by Christman B.W. et al. (Biomed. Environ. Mass Spectrom. 18, 258-264, 1989). However, as a result of diligent research on the cause of the rearrangement reaction, the present inventors have found that this isomerization is caused by a different step from those mentioned above, and have thus succeeded in minimizing the rearrangement reaction.

Specifically, the method of the invention sets the pH of the reaction solution to weak acidity for treatment of the PAF by phospholipase C. The present inventors were the first to discover that this condition is essential for suppressing the undesirable rearrangement reaction. The preferred weakly acidic pH range is approximately 4-7 and more preferably approximately 6. Treatment of PAF with phospholipase C may be carried out under conditions with, for example, at least 0.2 units, at 25-40°C for 15 minutes to 24 hours.

Since there are little contaminants of biological components in the purified sample obtained by step (3), the PAF can be hydrolyzed with a smaller amount of phospholipase C than conventional dose.

### (5) Removal of lysophosphatidylcholine (LysoPC)

The conditions for treating PAF with phospholipase C established in (4) above have the following technical significance. Specifically, the purified PAF obtained in (3) above may contain residual amounts of LysoPC that has not been sufficiently removed. In order to remove it, the reaction conditions were set based upon the difference in reactivities between PAF and LysoPC for phospholipase C, so that PAF is hydrolyzed whereas LysoPC is not done. Specifically, it has been a problem in the conventional methods that addition of ether (Ramesha, C.M. et al., Biomed. Environ. Mass Spectrom. 13, 107-111, 1986) to the reaction solution for hydrolysis of PAF also results undesirable in hydrolysis of LysoPC and the appearance of multiple interference peaks on selected ion recording gas chromatography. One of the features of the present invention is without using water-insoluble solvents such as ether which have hitherto been reported for accelerating hydrolysis of PAF.

In addition, a method has been established for separation of the glycerol compound from LysoPC in the reaction solution by using a reverse solid-phase extraction column for washing/elution with an acetonitrile/water mixture. The reverse-phase solid-phase extraction column used may be a commercially available product such as Bond Elut C18™. The preferred proportion range of acetonitrile/water for washing is 10/90-90/10, and as the elution solvent there may be used methanol, ethanol, acetonitrile, 1-propanol, tetrahydrofuran or the like.

### (6) Quantitation by GC-MS

### Derivatization for PAF

The above-mentioned PAF hydrolysate is converted to a pentafluorobenzoyl or other derivative and then purified with a normal solid-phase extraction column and analyzed by GC-MS. Numerous derivatizing agents are commercially available for GC-MS and there are no particular restrictions on their use, but pentafluorobenzoylating agents are preferred from the standpoint of sensitivity. A commercially available product may also be used for the normal reverse solid-phase extraction column.

### PAF analysis by gas chromatography

Since the sample prepared by the method described above has low inclusion of blood components and a high PAF yield, it allows detection of PAF by the highly sensitive negative ion/chemical ionization/electron capture/selected ion monotoring method, using a compact and inexpensive quadrupole mass spectrometer. The measurement precision can be significantly improved by utilizing a calibration curve constructed using an internal standard. It is particularly preferred to use a stable isotope labeled compound of PAF ([²H₄]PAF) as the internal standard.

The following are non-limitative examples of the GC-MS apparatuses, gas chromatography conditions and mass spectrometry conditions.

### Apparatus

Gas chromatograph: TraceGC (ThermoQuest)
Mass spectrometer: TraceMS (ThermoQuest)

### Gas chromatography conditions

Column: HP-1MS (30 m x 0.25 mm; membrane thickness: 0.25 µm; Hewlett-Packard Corp.)
Temperature programming mode: 150°C (2 min) -> 50°C/min -> 260°C (13 min) -> 50°C/min -> 310°C (5 min)
Injection port temperature: 300°C
Helium gas flow rate: 1.2 ml/min
Injection volume: 2 µl (splitless)
Injection cycle time: 22 min

### Mass spectrometry conditions

Ionization method: Negative ion/chemical ionization/electron capture
Measuring method: Selected ion monitoring
Reaction gas: methane
Monitoring ion: PAF; m/z 552, [²H₄]PAF; m/z 556
Multiplier voltage: 500 V
Transfer line temperature: 300°C
Ionization chamber temperature: 130°C

In order to verify the propriety of the method of the invention, and particularly the detection specificity, the present inventors extracted and purified PAF in samples by the method described above, subsequently treated it with recombinant human platelet-activating factor acetylhydrolase (hereinafter abbreviated as rhPAF-AH), and then confirmed whether or not the measured PAF was originated from the endogenous PAF in samples. The endogenous PAF concentrations in human blood by the method of the invention was found to be approximately 1 pg/mL, which is significantly lower than those of 10-30 pg/mL reported by Yamada, K. et al. in the only available report on this measurement by GC-MS (J. Chromatogr. 433, 243-247, 1988), demonstrating that the endogenous PAF concentration is extremely low. According to the invention, therefore, it has become possible for the first time to achieve a specific and highly-sensitive PAF level assay by the aforementioned procedures of extraction, purification and derivatization.

(6') The purified PAF from step (3) above may be used for the quantitation by a LC-MS method without being subjected to further pre-treatment.

The following are non-limitative examples of the LC-MS apparatuses, liquid chromatography conditions and mass spectrometry conditions.

### Apparatus

Liquid chromatograph: HP 1100 SERIES (Hewlett Packard)
Mass spectrometer: API 3000 (Applied Biosystems)

### Liquid chromatography conditions

Column: Inertsil SIL 150-5 (2.1 x 250 mm, GL Science)
Mobile phase: chloroform/methanol/10 mM ammonium acetate = 70/40/5
Flow rate: 0.2 mL/min
Injection volume: 20 µL

### Mass spectrometry conditions

Ionization by: electrospray ionization
Measurement by: selected reaction monitoring (SRM)
Monitoring ions: precursor negative ion -> product negative ion, PAF; m/z 508 -> m/z 59, [²H₄]PAF; m/z 512 -> m/z 59
Ion spray voltage: -4500 V
Orifice voltage : -91 V
Collision energy : -61 eV

### Examples

The present invention will now be explained in greater detail by way of the following examples, which are not intended to be limitative on the invention. The PAF and its labeling compounds used in the examples were as follows.
PAF: 1-O-Hexadecyl-2-acetyl-sn-glycero-3-phosphocholine
[³H]-PAF: 1-O-Hexadecyl-2-acetyl-sn-glycero-3-phosphocholine, [1-O-hexadecyl-1',2'-³H(N)]
[¹⁴C]-phosphatidylcholine (PC): Phosphatidylcholine, L-α-dipalmitoyl, [dipalmitoyl-1-¹⁴C]
[¹⁴C]-lysophosphatidylcholine (LysoPC): Lysopalmitoyl phosphatidyl choline, L-1-[Palmitoyl-1-¹⁴C]

### Example 1 Yield of PAF by total procedure

Table 1 shows the yields of PAF, PC and LysoPC through the total procedure, using human blood. After adding 1 mL of human blood to a tube containing 4 mL of tetrahydrofuran, [³H]PAF, [¹⁴C]-PC and [¹⁴C]-LysoPC were added and the mixture was stirred and centrifuged (3000 rpm x 15 min, 4°C). A 4 mL portion of ethyl acetate was added to the resulting supernatant and the mixture was stirred and centrifuged. The resulting supernatant was loaded into Bond Elut SI™, and then washed with 8 mL of hexane/2-propanol/water = 70/40/5 and eluted with 3 mL of hexane/2-propanol/water = 30/60/16. The eluted fraction was evaporated at 60°C under a nitrogen stream and the resulting residue was dissolved with 0.9 mL of 0.1 mM Bis-Tris buffer (pH 6.0, 10 mM CaCl₂, 0.1% Triton X-100), 0.1 mL of a phospholipase C solution (10 U/mL) was added, and the resulting mixture was incubated at 37°C for one hour. The reaction mixture was loaded into a Bond Elut 18™ column, and after washing the column with 8 mL of acetonitrile/purified water = 60/40, the elution was performed with 1 mL of acetonitrile. The sample was evaporated under a nitrogen stream, 0.1 ml of n-hexane solution containing pentafluorobenzoic anhydride solution (2 mg/mL of n-hexane solution) and 0.9 mL of 1.1% pyridine were added to the resulting residue, and the reaction mixture was allowed to stand at room temperature for one hour. The reaction solution was loaded into a Bond Elut SI™ column, and then washed with 8 mL of n-hexane/benzene = 60/40 and eluted with 3 mL of benzene. The eluate was evaporated under a nitrogen stream and the resulting residue was redissolved in 20 µL of ethyl acetate.

**Table 1**

| Yields of [³H]-PAF, [¹⁴H]-PC and [¹⁴C]-LysoPC through the total procedure steps | | | |
|---|---|---|---|
| Step | Total yield (%) | | |
| | [³H]-PAF | [¹⁴C]-PC | [¹⁴C]-LysoPC |
| Blood | 100 | 100 | 100 |
| Extraction with THF | 95(95) | 95(95) | 90(90) |
| Extraction with ethy acetate | 92(97) | 93(98) | 85(94) |
| Bond Elute SI (hexane/2-propanol/water =30/60/16 fraction) | 71(77) | 0(0) | 62(73) |
| Phospholipase C hydrolysis Bond Elute C18 (acetonitrile/purified water =60/40 fraction) | 50(70) | 0(-) | 0(0) |
| Pentafluorobenzoyl derivatization Bond Elute SI (n-hexane/benzene =60/40 fraction) | 45(90) | 0(-) | 0(-) |
| ( ): yield in each step | | | |

The PAF yield through the total procedure was found to be 45%. As shown in Table 1, PC was completely removed by the purification procedure with Bond Elut SI™, while LysoPC was completely removed by the purification procedure with Bond Elut C18™.

### Example 2 Selection of PAF extraction solvent

A 1 mL sample of human blood was added to each tube containing 4 mL of different solvents, and then [³H]-PAF was added before stirring. The sample was centrifuged (3000 rpm x 15 min, 4°C) and the radioactivity (³H-PAF) in the supernatant was measured, to give the results shown in Table 2.

The yield of [³H-PAF] in the supernatant was highest with tetrahydrofuran, followed by dioxane, acetonitrile and 2-propanol.

**Table 2**

| Yields of PAF with various solvents | |
|---|---|
| | [³H]-PAF yield (%) |
| Tetrahydrofuran | 98.1 ± 4.5 |
| Dioxane | 97.1 ± 3.1 |
| Acetonitrile | 95.1 ± 2.5 |
| 2-propanol | 93.2 ± 5.5 |
| Acetone | 85.3 ± 5.0 |
| Ethanol | 85.0 ± 3.3 |
| Methanol | 77.0 ± 4.0 |
| Mean ± SD (n=3) | |

### Example 3 Investigation of PAF peak specificity

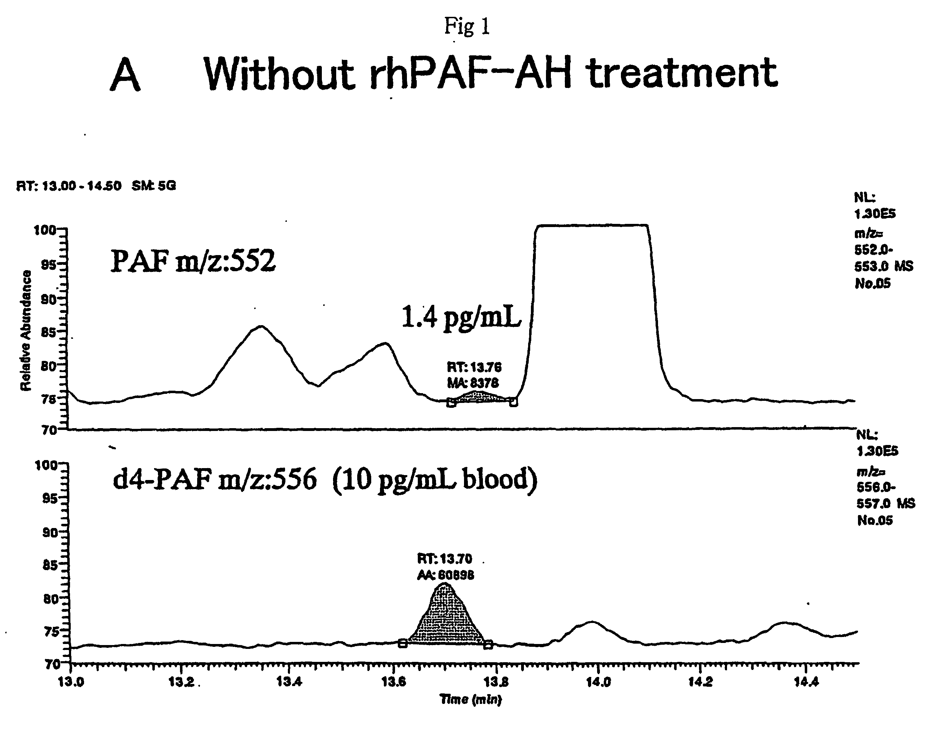

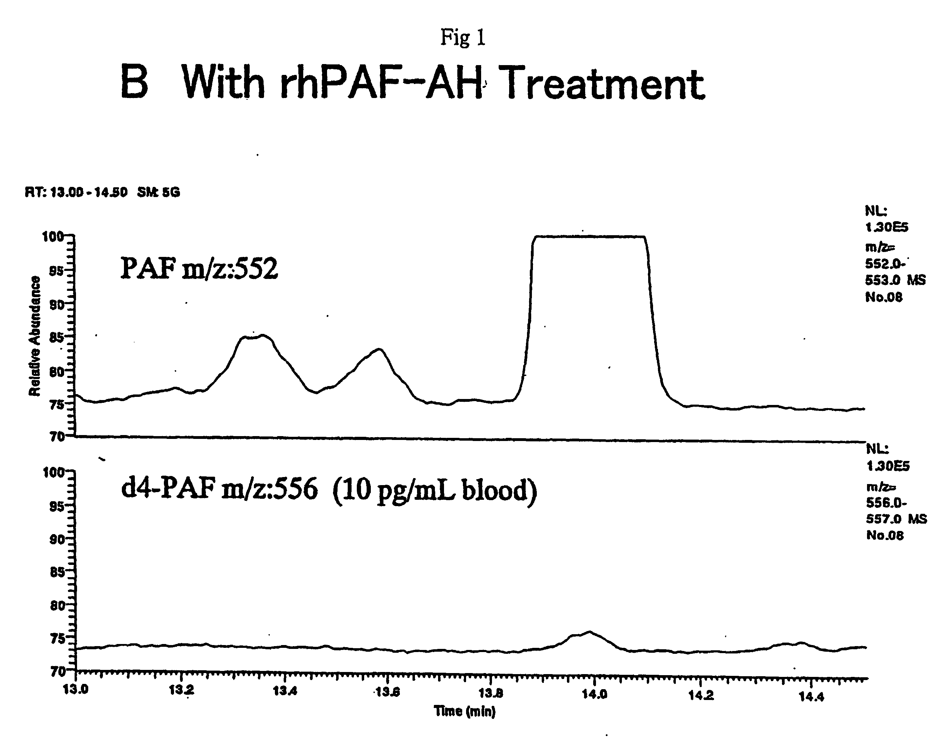

The uniformity of the PAF peak with gas chromatography was verified using rhPAF-AH, and the results are shown in Fig. 1. After extracting PAF from 1 mL of human blood using tetrahydrofuran and ethyl acetate according to the method described in Example 1, the resulting ethyl acetate extract was purified with Bond Elut SI™. The sample was dissolved in 0.5 mL of buffer solution containing 40 µg of rhPAF-AH, and the solution was incubated at 37°C for one hour. It was then treated according to the method described in Example 1, pentafluorobenzoylated, and subjected to analyzed by GC-MS.

For the sample untreated with rhPAF-AH, peaks for PAF and the internal standard ([²H₄]PAF) appeared at retention times of 13.76 minutes and 13.70 minutes, respectively, as shown in Fig. 1-A. For the sample treated with rhPAF-AH, however, these peaks had completely disappeared as shown in Fig. 1-B. These results indicated that the peak detected according to the present method is originated from PAF in the blood, and there was no biological components besides PAF.

### Example 4 Comparison between the invention method and an extraction method using methanol and chloroform

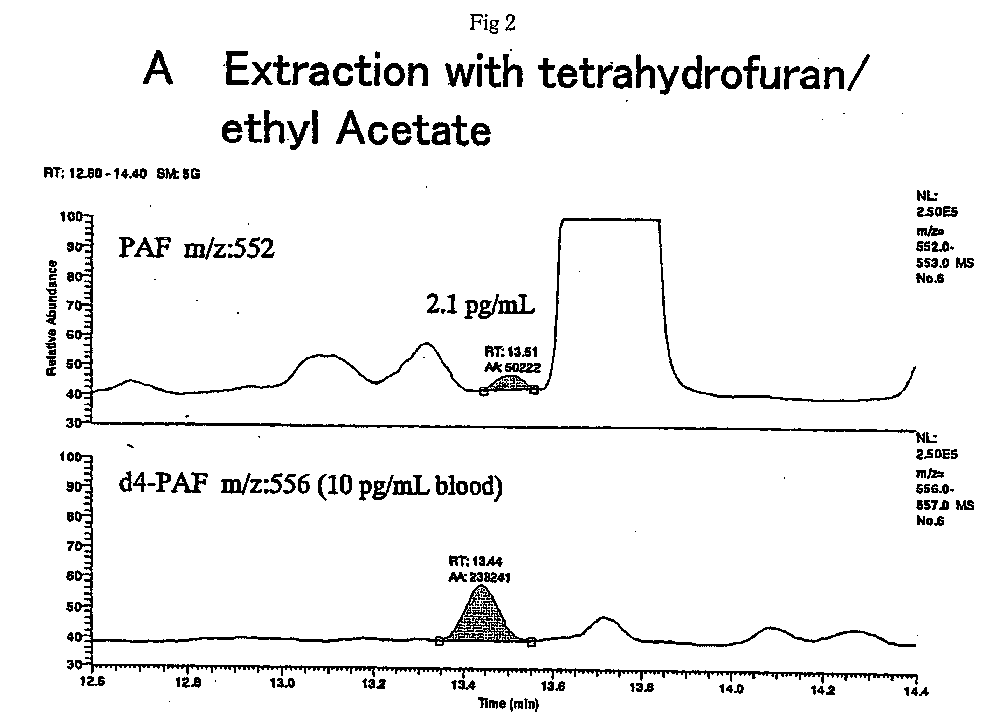

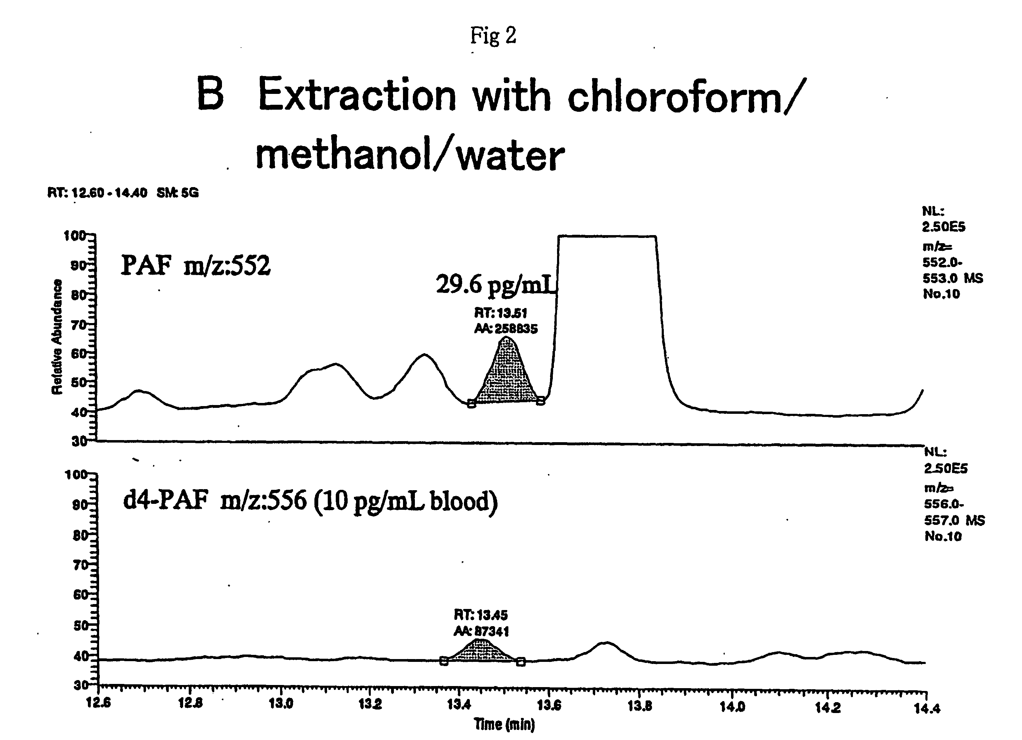

Fig. 2 shows the results of comparing the present invention method with a conventional method of Bligh & Dyer for extraction of PAF with a chloroform/methanol/water solution. The method was conducted in the same manner as Example 1 except for the conventional extraction step, which was as follows. A 1 mL sample of human blood was added to a tube containing 4 mL of methanol, and the mixture was stirred and centrifuged (3000 rpm x 15 min, 4°C). After adding 4 mL of chloroform and 1 mL of distilled water to the resulting supernatant, the mixture was again stirred and centrifuged. The resulted lower layer was purified with a Bond Elut SI™ according to the method described in Example 1, and followed by hydrolyzation with phospholipase C, pentafluorobenzoylation and analysis by GC-MS.

The human endogenous PAF blood level when measured by the method of the invention was found to be 2.1 pg/mL, as shown in Fig. 2-A. On the other hand, for a sample obtained by extraction of an identical human blood sample with a chloroform/methanol/water solution, the endogenous PAF blood level was 29.6 pg/mL, as shown in Fig. 2-B, thus clearly confirming that PAF and PAF analogs cannot be separated by the conventional method of Bligh & Dyer using a chloroform/methanol/water solution.

### Example 5 Comparison between the invention method and a conventional method for purification by normal solid-phase column

The method of the invention was compared with the method of Weintraub, S.T. et al. (J. Am. Soc. Mass Spectrom. 11, 176-181, 2000) in regard to the purification step with a normal solid-phase column. Weintraub, S.T. et al. reported PAF concentration assay in cultured cells by their method in which PAF is extracted from cultured cells using methanol and chloroform, and the extract was loaded onto a normal solid-phase column and then washed with n-hexane/2-propanol/water = 46.75/46.75/6.5 followed by eluting with chloroform/methanol/water = 1/2/0.8.

In order to compare the present invention with the purification in a normal solid-phase column as described in this publication, human blood identical to that of Example 4 was used as the sample, and the entire procedure was performed in the same manner as Example 1 except for using the washing solution and eluting solution used in the normal solid-phase column purification step. The washing solution used in the normal solid-phase column for purification step was n-hexane/2-propanol/water = 46.75/46.75/6.5 and the eluting solution was chloroform/methanol/water = 1/2/0.8.

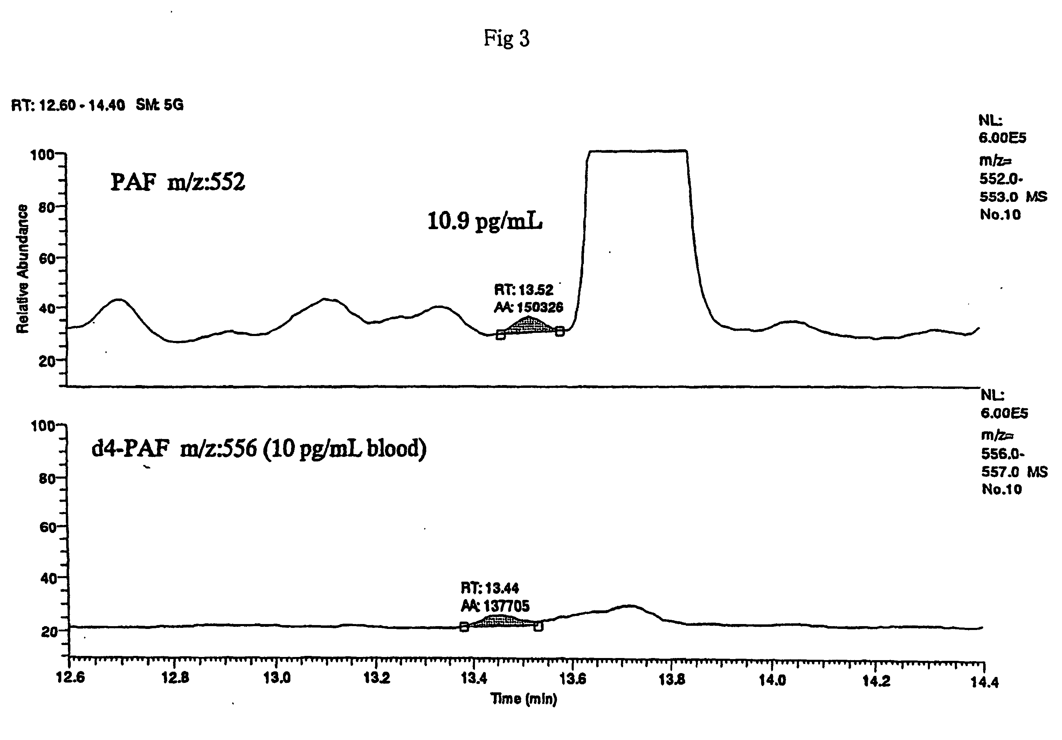

As shown in Fig. 3, the human endogenous PAF blood level was found to be 10.9 pg/mL by the method of Weintraub, S.T. et al. Since this result was much higher than that of 2.1 pg/mL shown in Fig. 2A of Example 4, or the average human endogenous PAF concentration of 1.1 pg/mL (n=21) mentioned below in Example 7, demonstrating that PAF and PAF analogs cannot be separated under the normal solid-phase column using washing and eluting conditions according to the method of Weintraub, S.T. et al.

### Example 6 PAF isomer production inhibition

Fig. 4 shows the reaction conditions for inhibiting the production of PAF isomers when PAF is hydrolyzed with phospholipase C. PAF was dissolved in buffer solutions (containing 100 mM Bis-Tris or 100 mM HEPES, 10 mM CaCl₂ and 0.1% Triton X-100) at pH 6.0, 7.0, 7.5 and 8.0, and in the same manner as Example 1, was subjected to hydrolysis with phospholipase C, pentafluorobenzoylated and analyzed by GC-MS.

As shown in Fig. 4-A, no PAF isomer was produced in the reaction solution at pH 6.0, but it increased with higher pH. The PAF peak area reached to the maximum at pH 6.0, and decreased with higher pH. It was thus shown that isomer is produced in the conventional pH range (7.5-8.0) of phospholipase C hydrolyzing solutions, resulting the decrease of the peak intensity. By the method of the invention, however, it was possible to inhibit rearrangement reaction by setting the pH of the reaction solution to weak acidity.

### Example 7 Construction of calibration curve

After adding PAF and an internal standard ([²H₄]PAF) to human blood at the concentrations of 0-181 pg/mL and 10 pg/mL, respectively the samples at each concentration were assayed with triplicated runs by the method described in Example 1.

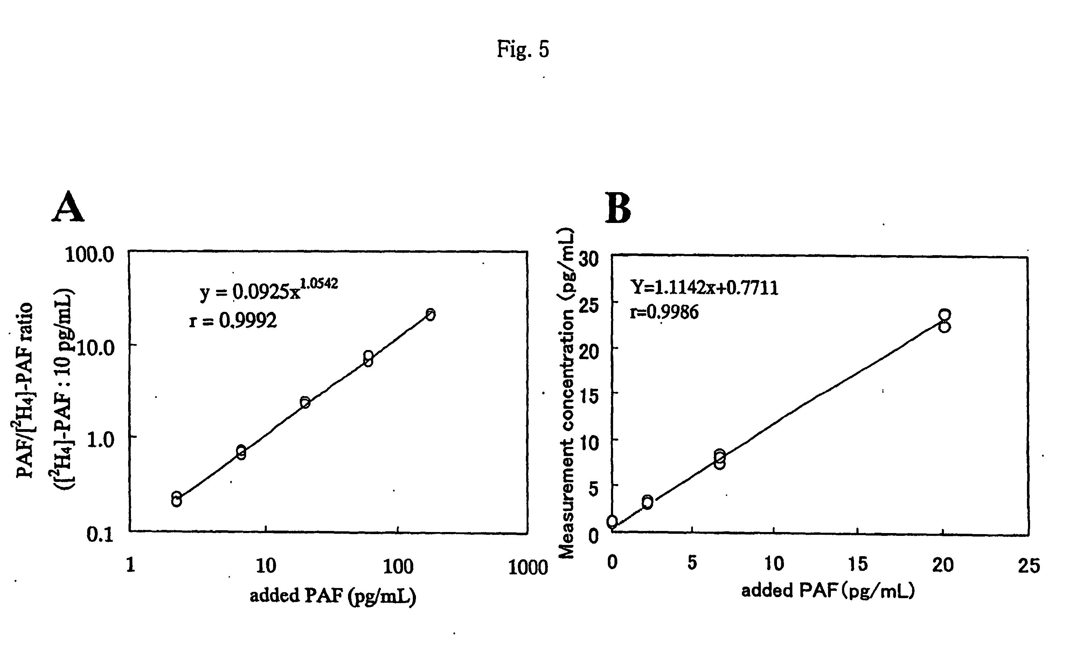

As shown in Fig. 5-A, the correlation coefficient of the calibration curve was calculated to be 0.9992, and a satisfactory linearity was obtained in the range of 0-181 pg/mL. The coefficients of variation (CV) for measurement at each concentration were small in the range of 3.7-11.4%. The mean endogenous human PAF blood level and standard deviation measured at this time was found to be 1.1 ±1.0 pg/mL (n=21).

PAF was added to human blood in the range of 0-20.2 pg/mL and a calibration curve was constructed. As shown in Fig. 5-B, the regression equation for this case is represented by Y = 1.114 x the spiked PAF concentration + 0.7711; therefore the estimated endogenous PAF blood level was extrapolated to be 0.77 pg/ml when the spiked amount (x) of PAF was zero. The extrapolated level was practically agreed with the endogenous PAF blood level of 1.1 pg/mL. This further substantiated the propriety of the assay values of the present inventors.

### Example 8 Application of this invention method to liquid chromatography-mass spectrometry

The PAF extraction and purification method described in Example 1 was applied to liquid chromatography-mass spectrometry (LC-MS) which allows direct analysis without derivatization of PAF. A 1 mL portion of human blood was added to a tube containing 4 mL of tetrahydrofuran. An internal standard of 50 pg/mL ([²H₄]PAF) was added to a blood, and this was followed by stirring and centrifugation (3000 rpm x 15 min, 4°C) After adding 4 mL of ethyl acetate to the resulting supernatant, the mixture was stirred and centrifuged. The resulted supernatant was loaded into a Bond Elut SI™ column, washed with 8 mL of hexane/2-propanol/water = 70/40/5 and eluted with 3 mL of hexane/2-propanol/water = 30/60/16. The eluted fraction was evaporated at 60°C under a nitrogen stream and the residue was dissolved with 50 µL of an HPLC mobile phase (chloroform/methanol/10 mM ammonium acetate = 70/40/5). A 20 pL portion of the sample was introduced into the following LC-MS System. An HP1100 SERIES (Hewlett Packard) was used for HPLC, with Inertsil SIL 150-5 (2.1 x 250 mm, GL Science) as the column and chloroform/methanol/10 mM ammonium acetate = 70/40/5 as the mobile phase, at a flow rate of 0.2 mL/min. The mass spectrometry was carried out using an API3000 (Applied Biosystems), and ionization was effected by with a turbo ion spray system. The selected reaction monitoring (SRM) was carried out using the precursor negative ion and product negative ion at m/z values of 508 and 59 for PAF and 512 and 59 for [²H₄]PAF.

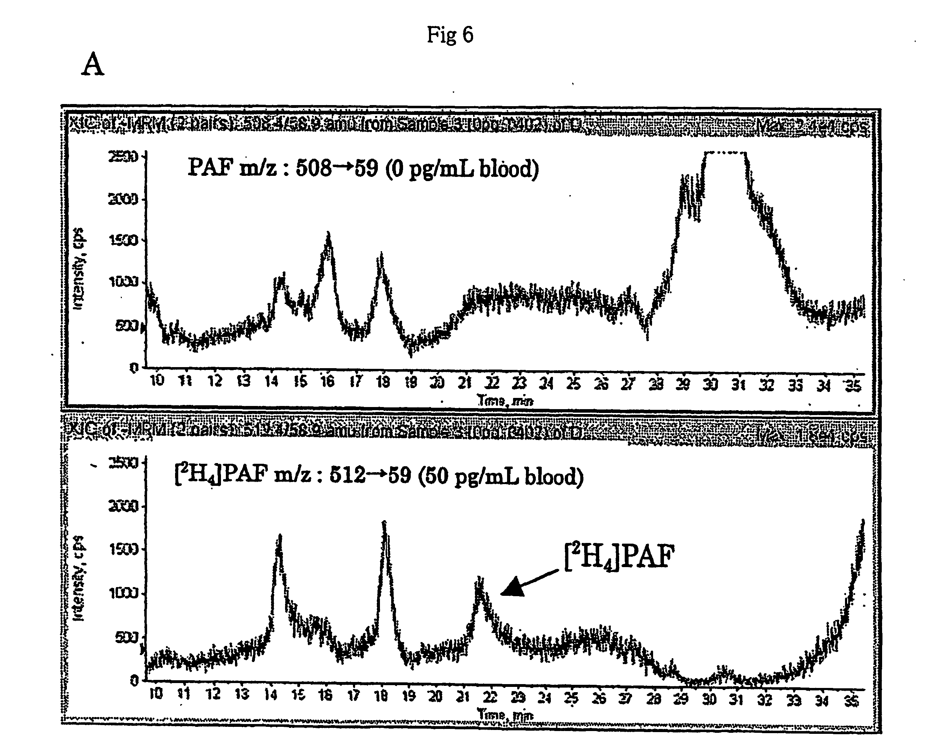

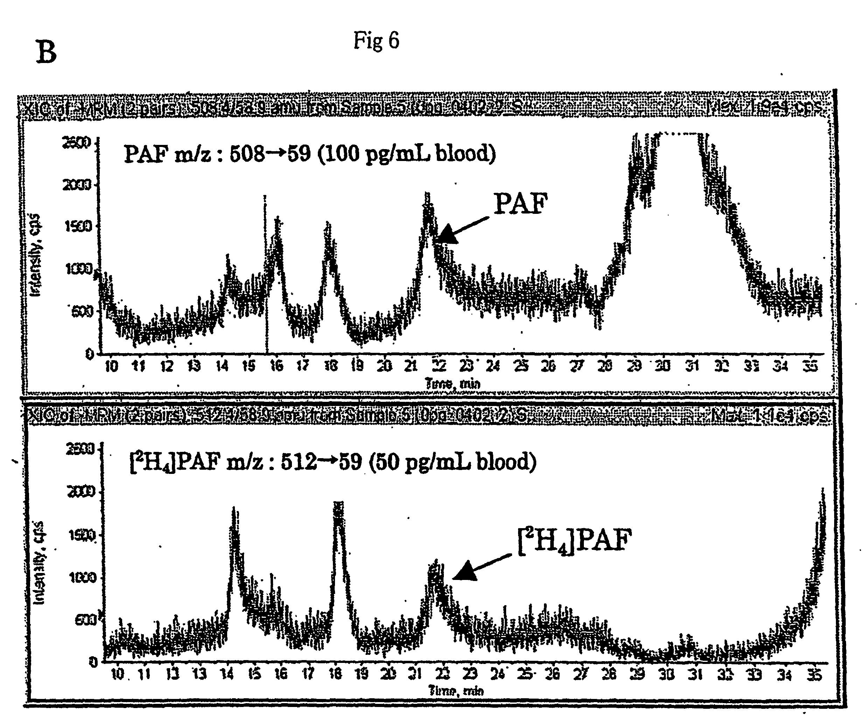

As shown in Fig. 6-A, a [²H₄]PAF peak was detected at a retention time of 21.5 min for the sample obtained by adding 50 pg/mL of [²H₄]PAF to blood, and there were no blood contaminant peaks at that retention time. On the other hand, as shown in Fig. 6-B, the peaks for PAF and [²H₄]PAF were detected for the samples obtained by adding 100 pg/mL of PAF and 50 pg/mL of [²H₄]PAF to blood. These results indicated that blood PAF can be assayed even by LC-MS/MS utilizing the extraction and purification method of the invention.

### EFFECT OF THE INVENTION

It has been shown that the method of the invention allows specific and precise measurement of PAF concentrations in biological samples. It has been pointed out that the PAF assay is difficult in the prior art due to the coexistence of large amounts of phospholipids with a structure similar to PAF in biological samples, and due to very low PAF levels in the body. The present inventors have completed the present invention upon discovering a method for selective extraction and purification of PAF from biological samples, and establishing a highly sensitive PAF assay method with excellent reproducibility and reliance. The present invention may be employed to measure PAF levels in biological samples for elucidating the role of PAF in various pathological conditions. In addition, it is expected to facilitate diagnosis of various diseases associated with PAF fluctuation, and also contributing to evaluation of therapeutic effects on such diseases.

## Claims

1. A method of treating a biological sample in order to measure the quantity of platelet-activating factor (PAF) represented by the following formula: 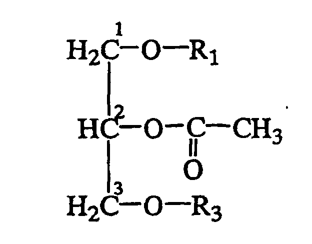 where R₁ is a saturated or unsaturated noncyclic hydrocarbon group or an acyl group derived from a saturated or unsaturated fatty acid, and R₃ is phosphocholine, phosphoethanolamine, phosphomonomethylethanolamine or phosphodimethylethanolamine,
contained in said biological sample, the method comprising the steps of
a) mixing the biological sample with a water-soluble organic solvent and separating the mixture by centrifugation,
b) extracting the PAF contained in the supernatant obtained from said centrifugation with a water-insoluble organic solvent, and
c) introducing the PAF extracted in the water-insoluble organic solvent onto a silica gel solid-phase column either directly or after adjustment to a suitable concentration, and performing washing and elution with a mixture of hexane/2-propanol/water to purify the PAF in biological samples.

2. The method of claim 1, wherein the water-soluble organic solvent of step a) is tetrahydrofuran.

3. The method of claim 1 or 2, wherein the water-insoluble organic solvent of step b) is ethyl acetate.

4. The method of any one of claims 1 to 3, wherein the washing of step c) is carried out with a mixture of hexane/2-propanol/water at 1/0.5-1/0.05-0.2.

5. The method of claim 4, wherein the elution of step c) is carried out with a mixture of hexane/2-propanol/water at 1/2-20/0.2-2.

6. The method of any one of claims 1 to 5, wherein the method of measuring the quantity of PAF is gas chromatography-mass spectrometry.

7. The method of claim 6, which comprises the additional steps of d) hydrolyzing the PAF purified in step c) with phospholipase C and e) purifying the resulted PAF hydrolysate (hereunder referred to as glycerol compound).

8. The method of claim 7, wherein the hydrolysis with phospholipase C is conducted under weakly acidic conditions.

9. The method of claim 7 or 8, wherein the purification of the glycerol compound is accomplished by washing and elution with a reverse-phase silica gel column using an acetonitrile/water mixture.

10. A method for measuring the quantity of PAF in a biological sample wherein after the purification of the glycerol compound by step e) according to any one of claims 7 to 9 it is subjected to a step of f) converting it to a derivative suitable for gas chromatography-mass spectrometry, with purification of said derivative if necessary, separating it by gas chromatography and subjecting the separated components to mass spectrometry.

11. The method of claim 10, which further comprises a step of quantifying the PAF quantity of the biological sample by comparing the measured values obtained from the biological sample with a calibration curve obtained by conducting steps a) to f) by addition of known amounts of a PAF standard sample in the same manner as for the biological sample.

12. The method of any one of claims 1 to 5, wherein the method of measuring the PAF quantity is liquid chromatography-mass spectrometry.

13. The method of claim 12, which further comprises a step of determining the PAF content of the biological sample by comparing the measured values obtained for the biological sample with a calibration curve obtained by addition of known amounts of a PAF standard sample in the same manner as for the biological sample.

## Patentansprüche

1. Verfahren zur Behandlung einer biologischen Probe, um die Quantität von thrombozytenaktivierendem Faktor (PAF), dargestellt durch die folgende Formel: 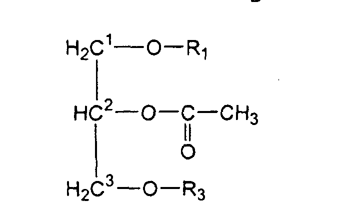 worin R₁ eine gesättigte oder ungesättigte nichtcyclische Kohlenwasserstoffgruppe oder eine Acylgruppe ist, abgeleitet von einer gesättigten oder ungesättigten Fettsäure, und R₃ ist Phosphocholin, Phosphoethanolamin, Phosphomonomethylethanolamin oder Phosphodimethylethanolamin,
enthalten in der biologischen Probe, zu messen, wobei das Verfahren die folgenden Schritte umfaßt:
a) Vermischen der biologischen Probe mit einem wasserlöslichen organischen Lösungsmittel und Abtrennen der Mischung durch Zentrifugation,
b) Extraktion des PAF, enthalten in dem Überstand, erhalten durch die Zentrifugation mit einem wasserunlöslichen organischen Lösungsmittel und
c) Einführen des PAF, extrahiert in dem wasserunlöslichen organischen Lösungsmittel, auf eine Silicagel-Festphasensäule, entweder direkt oder nach Einstellung auf eine geeignete Konzentration, und Durchführung des Waschens und der Elution mit einer Mischung aus Hexan/2-Propanol/Wasser, um den PAF in biologischen Proben zu reinigen.

2. Verfahren gemäß Anspruch 1, wobei das wasserlösliche organische Lösungsmittel in Schritt a) Tetrahydrofuran ist.

3. Verfahren gemäß Anspruch 1 oder 2, wobei das wasserunlösliche organische Lösungsmittel von Schritt b) Ethylacetat ist.

4. Verfahren gemäß einem der Ansprüche 1 bis 3, wobei das Waschen gemäß Schritt c) mit einer Mischung aus Hexan/2-Propanol/Wasser in einem Verhältnis von 1/0,5-1/0,05-0,2 durchgeführt wird.

5. Verfahren gemäß Anspruch 4, wobei die Elution gemäß Schritt c) mit einer Mischung aus Hexan/2-Propanol/Wasser in einem Verhältnis von 1/2-20/0,2-2 durchgeführt wird.

6. Verfahren gemäß einem der Ansprüche 1 bis 5, wobei das Verfahren zur Messung der Quantität von PAF eine Gaschromatografie-Massenspektrometrie ist.

7. Verfahren gemäß Anspruch 6, das die zusätzlichen Schritte von d) Hydrolyse des in Schritt c) gereinigten PAF mit Phospholipase C und e) Reinigung des resultierenden PAF-Hydrolysats (hiernach bezeichnet als Glycerinverbindung) umfaßt.

8. Verfahren gemäß Anspruch 7, wobei die Hydrolyse mit Phospholipase C unter schwach sauren Bedingungen durchgeführt wird.

9. Verfahren gemäß Ansprüchen 7 oder 8, wobei die Reinigung der Glycerinverbindung durch Waschen und Elution mit einer Umkehrphasen-Silicagelsäule unter Verwendung einer Acetonitril/Wasser-Mischung bewirkt wird.

10. Verfahren zur Messung der Quantität von PAF in einer biologischen Probe, wobei nach der Reinigung der Glycerinverbindung durch Schritt e) gemäß einem der Ansprüche 7 bis 9 sie einem Schritt f), nämlich Umwandlung in ein Derivat, das für die Gaschromatografie-Massenspektrometrie geeignet ist, mit Reinigung des Derivats falls nötig, Abtrennung durch Gaschromatografie und Bewirken einer Massenspektrometrie an den abgetrennten Komponenten, unterzogen wird.

11. Verfahren gemäß Anspruch 10, das weiterhin den Schritt der Quantifizierung der PAF-Menge der biologischen Probe durch Vergleich der gemessenen Werte, erhalten von der biologischen Probe, mit einer Kalibrierungskurve, erhalten durch Durchführung der Schritte a) bis f) durch Zugabe von bekannten Mengen einer PAF-Standardprobe in derselben Weise für die biologische Probe umfaßt.

12. Verfahren gemäß einem der Ansprüche 1 bis 5, wobei das Verfahren zur Messung der PAF-Menge eine Flüssigchromatografie-Massenspektrometrie ist.

13. Verfahren gemäß Anspruch 12, das weiterhin den Schritt der Bestimmung des PAF-Gehalts der biologischen Probe durch Vergleich der gemessenen Werte, erhalten für die biologische Probe, mit einer Kalibrierungskurve, erhalten durch Zugabe von bekannten Mengen einer PAF-Standardprobe in derselbe Weise wie für die biologische Probe umfaßt.

## Revendications

1. Procédé pour traiter un échantillon biologique afin de mesurer la quantité de facteur d'activation plaquettaire (PAF) représenté par la formule suivante : 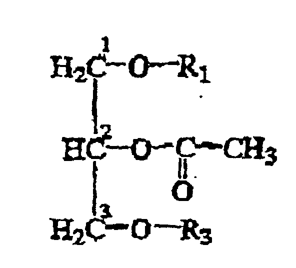 où R₁ est un groupe hydrocarboné saturé ou insaturé non cyclique ou un groupe acyle dérivé d'un acide gras saturé ou insaturé, et R₃ est de la phosphocholine, de la phosphoéthanolamine, de la phosphomonométhyléthanolamine ou de la phosphodiméthyléthanolamine, contenu dans ledit échantillon biologique, le procédé comprenant les étapes consistant à
a) mélanger l'échantillon biologique avec un solvant organique hydrosoluble et à séparer le mélange par centrifugation,
b) extraire le PAF contenu dans le surnageant obtenu à partir de ladite centrifugation avec un solvant organique insoluble dans l'eau, et
c) introduire le PAF extrait dans le solvant organique insoluble dans l'eau dans une colonne en phase solide de gel de silice soit directement soit après ajustement à une concentration convenable, et à réaliser le lavage et l'élution avec un mélange hexane/2-propanol/eau pour purifier le PAF dans les échantillons biologiques.

2. Procédé selon la revendication 1, dans lequel le solvant organique hydrosoluble de l'étape a) est le tétrahydrofurane.

3. Procédé selon la revendication 1 ou 2, dans lequel le solvant organique insoluble dans l'eau de l'étape b) est de l'acétate d'éthyle.

4. Procédé selon l'une quelconque des revendications 1 à 3, dans lequel le lavage de l'étape c) est réalisé avec un mélange hexane/2-propanol/eau à 1/0,5 à 1/0,05 à 0,2.

5. Procédé selon la revendication 4, dans lequel l'élution de l'étape c) est réalisée avec un mélange hexane/2-propanol/eau à 1/2 à 20/0,2 à 2.

6. Procédé selon l'une quelconque des revendications 1 à 5, dans lequel le procédé pour mesurer la quantité de PAF est la chromatographie en phase gazeuse couplée à la spectrométrie de masse.

7. Procédé selon la revendication 6, qui comprend les étapes supplémentaires consistant à d) hydrolyser le PAF purifié à l'étape c) avec de la phospholipase C et e) purifier l'hydrolysat de PAF ainsi obtenu (ci-après nommé composé glycérol).

8. Procédé selon la revendication 7, dans lequel l'hydrolyse par la phospholipase C est réalisée dans des conditions faiblement acides.

9. Procédé selon la revendication 7 ou 8, dans lequel la purification du composé glycérol est réalisée par lavage et élution avec une colonne de gel de silice en phase inverse en utilisant un mélange acétonitrile/eau.

10. Procédé pour mesurer la quantité de PAF dans un échantillon biologique dans lequel après la purification du composé glycérol par l'étape e) selon l'une quelconque des revendications 7 à 9, il est soumis à une étape consistant à f) le convertir en un dérivé adapté à la chromatographie en phase gazeuse - couplée à la spectrométrie de masse, avec purification dudit dérivé si nécessaire, en le séparant par chromatographie en phase gazeuse et en soumettant les composants séparés à une spectrométrie de masse.

11. Procédé selon la revendication 10, qui comprend en outre une étape consistant à quantifier la quantité de PAF de l'échantillon biologique en comparant les valeurs mesurées obtenues à partir de l'échantillon biologique avec une courbe d'étalonnage obtenue par réalisation des étapes a) à f) par ajout de quantités connues d'un échantillon standard de PAF de la même manière que pour l'échantillon biologique.

12. Procédé selon l'une quelconque des revendications 1 à 5, dans lequel le procédé pour mesurer la quantité de PAF est la chromatographie en phase liquide couplée à la spectrométrie de masse.

13. Procédé selon la revendication 12, qui comprend en outre une étape consistant à déterminer la teneur en PAF de l'échantillon biologique en comparant les valeurs mesurées obtenues pour l'échantillon biologique avec une courbe d'étalonnage obtenue par ajout de quantités connues d'un échantillon standard de PAF de la même manière que pour l'échantillon biologique.
